Europäisches Patentamt

European Patent Office

Office européen des brevets

(11) Publication number: **0 339 821**
**A1**

## (12) EUROPEAN PATENT APPLICATION

(21) Application number: 89303528.7

(22) Date of filing: 11.04.89

(51) Int. Cl.⁴: **G01N 33/553 , G01N 33/543 , G01N 33/547**

(30) Priority: 21.04.88 GB 8809429

(43) Date of publication of application:
**02.11.89 Bulletin 89/44**

(84) Designated Contracting States:
**DE FR NL**

(71) Applicant: **UNITED KINGDOM ATOMIC
ENERGY AUTHORITY**
**11 Charles II Street**
**London SW1Y 4QP(GB)**

(72) Inventor: **Chadwick, Andrew Thomas**
**Pentrose Fieldside**
**Upton nr Didcot Oxfordshire(GB)**
Inventor: **Hudson, Michael James**
**18 Cypress Road Woodley**
**Reading Berkshire RG5 4BD(GB)**

(74) Representative: **Mansell, Keith Rodney et al**
**United Kingdom Atomic Energy Authority**
**Patents Branch B329 Harwell Laboratory**
**Oxfordshire OX11 0RA(GB)**

(54) **Bonding to metal surfaces.**

(57) Metal surfaces have chemical species, e.g. biologically active water-soluble macromolecular substances such as proteins, bonded thereto by means of an interposed coordinating polymer. The polymer has a first set of functional groups bonded to the surface and a second set of functional groups bonded to the species; the groups of the first set are pendant from the polymer chain or form part thereof, each such group preferably containing an atom with electrons for donation to the metal surface to form the bond therewith. The metal may be a transition metal such as a Group 11 metal, e.g. Au or Ag, and, in the coordinating polymer, the sets may comprise functional groups that are the same or different chemically, e.g. the first set may comprise S-containing groups and the second set amino groups. A specific example of co-ordinating polymer that may be used is a water soluble form of poly-(iminoethene)N-dithiocarboxylate. Metal surfaces so treated may be useful as biosensor materials such as in immunosensors.

## Bonding to Metal Surfaces

This invention relates to metal surfaces having chemical species such as biologically active substances bonded thereto by means of an interposed coordinating polymer.

There is a need to attach biologically active substances to metal surfaces to give the surface some specific affinity, for example to make biosensor materials such as for use in the design of immunosensors, or in the production of immunogold reagents in agglutination immunoassay or in immunohistology. The present invention is concerned with attaching such substances and other chemical species to metal surfaces.

In one aspect, the present invention provides a metal surface having a chemical species bonded thereto by means of an interposed coordinating polymer, the polymer having a first set of functional groups bonded to the surface and having a second set of functional groups bonded to the species, the groups of the first set being pendant from the polymer chain or forming part thereof, each such group preferably containing an atom with electrons for donation to the metal surface to form the bond therewith.

In a second aspect, the present invention provides a method of bonding a chemical species to a metal surface comprising the steps of

(i) bonding a coordinating polymer to a metal surface, the polymer having a first set of functional groups capable of bonding to the surface and having a second set of functional groups capable of bonding to the species, the groups of the first set being pendant from the polymer chain or forming part thereof, each such group preferably containing an atom with electrons for donation to the metal surface to form the bond therewith, and the second set being such that the groups thereof do not bond to the metal surface; and

(ii) bonding the chemical species to the polymer via the second set of functional groups.

It has been found that the present invention enables chemical species, e.g active chemical species such as biologically active substances, to be securely and permanently attached to metal surfaces of artefacts. Where the species is active, it is found that its activity may be retained; such artefacts may therefore be used in the above-mentioned applications, e.g. as components of biosensors.

The species may be bonded directly or indirectly to the second set of functional groups, e.g. in the latter case it may be bonded via a linking reagent.

Examples of suitable metals in the practice of the invention are transition metals such as precious metals (e.g. Au, Ag and the Pt metals) and base metals (e.g. Cu, Fe, Ni and Cr). Metals of Group 11 and 12 (formerly referred to as Groups 1B and IIB) of the Periodic Table are preferred.

The coordinating polymer may be a homopolymer or a copolymer; also it may be an oligomer. Preferably, there is little or no cross-linking in the polymer. The polymer may, for example, be a polyimine or a polythiol. Also, the functional groups of the first set in the polymer may be chemically the same as or different from the functional groups of the second set. Where they are different, the groups of the first set may be S-containing groups and the groups of the second set amino groups: a particular example of a coordinating polymer having such groups is poly-(iminoethene)N-dithiocarboxylate, referred to herein for convenience as "PIED".

The first set of functional groups may bond to the metal surface by being chemisorbed thereon by means of a ligand-metal interaction, which may be referred to as a ligating interaction, the bond formed possibly being covalent or having a significant degree of covalent character. The polymer may form a film on the metal surface. The stability of the bond between the metal surface and the polymer is enhanced by the "chelate" or polymer effect since each molecule of the polymer possesses more than one functional group for bonding thereto. This effect particularly occurs in polymers having little or no cross-linking.

The ability of the functional groups in the first set of functional groups to bond to the metal surface and the inability of the functional groups in the second set of functional groups to bond to the metal surface are influenced by thermodynamic, conformational and/or other factors such as the surface area of the metal and the total concentration of functional groups in the first set on the polymer. Preferably, there are more functional groups in the first set than there are available sites for bonding on the metal surface.

Usually, the functional groups of a particular set are chemically all the same. However, this need not necessarily be so.

Where the species is an active chemical species, the activity may be biological activity as indicated hereinbefore but is not necessarily so restricted. For example, the activity may be electrocatalytic or chromatographic. The species may be inactive, for example to provide an inert surface layer over the metal.

The chemical species may be molecular or atomic, and may be organic, organometallic or inorganic. It may be charged or electrically neutral, or it may be a radical. Preferably, it is a biologically active substance comprising organic molecules, for example sensor molecules for use in immunosensing. Specific examples of such biologically active substances are water-soluble macromolecular substances such as proteins, e.g. antibodies, and affinity reagents.

Clearly, the scope of the present invention is limited by the ability of the constituent materials - the metal surface, the coordinating polymer and the chemical species - to form stable bonds with one another as required by the invention. Thus, experiments may have to be carried out to determine whether particular combinations of constituent materials are suitable. For example, if the groups of the first set in the polymer are in the polymer backbone, the polymer must have sufficient conformational flexibility to enable at least some of them to bond to the metal atoms or ions at the metal surface.

The coordinating polymer may be soluble or partially soluble in a solvent which does not react with the metal or any support thereof. Thus, the method of the present invention is conveniently carried out by, in step (i), contacting the surface with a solution of the coordinating polymer in such a solvent, for example in dilute (e.g. 1%) solution. Where the coordinating polymer is water-soluble, the solution may be aqueous.

In the method, the groups of the first set bond to the metal surface and, where said groups are oxidisable, for example they comprise -SH, they are able to displace any thin layer of oxide or tarnish that may have formed thereon. PIED dissolved in water was found to give a rapidly formed layer of significant electrical resistance over the metal surface.

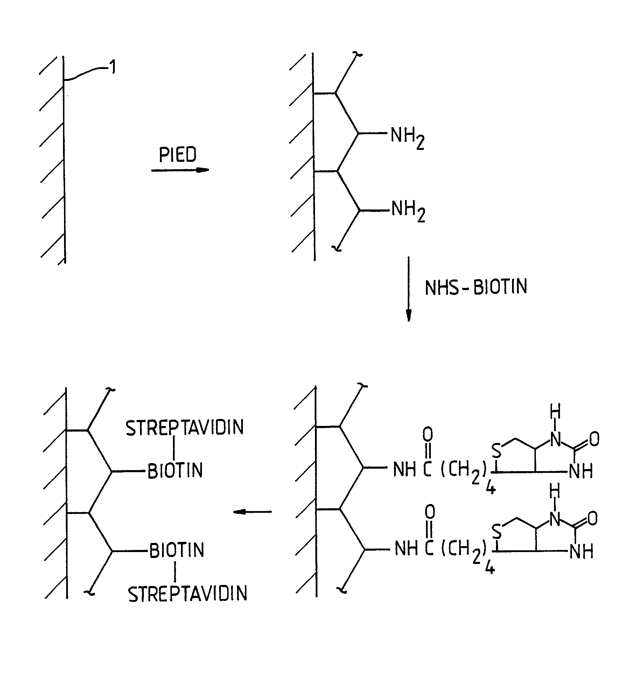

The following examples illustrate the invention; initials will be used from time to time to represent lengthy names as will be apparent in the text. Reference will be made to the accompanying drawing, the sole figure of which is a reaction scheme for bonding organic molecules to a metal surface according to the invention. In the examples, all components are in 0.1 M NaCl aqueous solution buffered with 0.1 M phosphate at pH 7.

EXAMPLE 1

Preparation of Coated Electrode

A bare gold electrode (4 mm thickness) was contacted with an aqueous solution of poly-(iminoethene)N-dithiocarboxylate (PIED) for 10 minutes. It is estimated that 1% of nitrogen-containing functional groups were present as N-dithiocarboxylate in this material. It should be noted that, with higher percentages, the polymer is essentially insoluble.

The electrode capacitance was measured over this period and was observed to fall substantially (from 96.0 $\mu F/cm^2$ to 13.4 $\mu F/cm^2$) thereby showing that the PIED had been sorbed on the electrode surface.

The treated electrode was then reacted with biotin (a vitamin) using its N-hydrosuccinimodo derivative (NHS-biotin). The reaction time was again 10 minutes and a further fall in electrode capacitance was observed.

Testing of Coated Electrode

The electrode was treated with N-ethylmaleimide (NEM) to block any remaining amino groups on the PIED surface and finally treated with fluorescently labelled streptavidin which bonded strongly to the biotin. Fluorescence was detected at the electrode surface microscopically.

Referring to the figure, in the first reaction, the sulphur-containing groups in the PIED bind to the metal surface 1 leaving at least some of the amino groups free. In the second reaction, the free amino groups react with the biotin and in the third reaction, the biotin reacts with the streptavidin. The blocking of the remaining amino groups by NEM between the second and third reactions is not shown.

EXAMPLE 2

Preparation of Coated Electrode

A gold electrode as used in Example 1 was contacted with a polythiol, a poly(thioethene) matrix containing a large number of thiol groups, for 10 minutes. The electrode capacitance fell from 101.6 $\mu F/cm^2$ to 17.28 $\mu F/cm^2$ over that period as the polythiol became bound to the electrode surface via its thiol groups.

The treated electrode was then reacted with succinimidyl-4-(N-maleimidomethyl)cyclohexane-1-carboxylate (SMCC) which reacted with remaining thiol groups in the bonded polythiol via its maleimide moiety.

The electrode was next treated with rabbit IgG, the amine groups of which reacted with the succinimidyl residue of the SMCC.

Testing of Coated Electrode

The coated electrode was treated with bovine-serum albumen (BSA) and then anti-rabbit IgG. The BSA was used to prevent the anti-rabbit IgG from binding to unused SMCC. Reaction with the antibody was shown by fluorescence microscopy.

EXAMPLE 3

The procedure of Example 2 was repeated with the exceptions that PIED was used as the co-ordinating polymer instead of polythiol and glutaraldehyde used as the cross-linking agent instead of SMCC.

As in Example 2, adsorption of the co-ordinating polymer was detected by observing a reduction in electrode capacitance and reaction with the antibody shown by fluorescence microscopy.

In all three of the above examples, it is shown that the organic molecules retain their biological activity when coated onto a gold electrode by means of a co-ordinating polymer.

**Claims**

1. A metal surface having a chemical species bonded thereto by means of an interposed polymer, characterised in that the polymer is a co-ordinating polymer having a first set of functional groups bonded to the surface and having a second set of functional groups bonded to the species, the groups of the first set being pendant from the polymer chain or forming part thereof.

2. A metal surface according to claim 1 wherein the metal is a metal of Group II (formerly 1B) of the Periodic Table.

3. A metal surface according to either of the preceding claims wherein the coordinating polymer is a polyimine or a polythiol.

4. A metal surface according to any of the preceding claims wherein the groups of the first set are S-containing groups and the groups of the second set are amino groups.

5. A metal surface according to claim 4 wherein the coordinating polymer is poly-(iminoethene)N-dithiocarboxylate.

6. A metal surface according to any of the preceding claims wherein the chemical species is a biologically active, water-soluble macromolecular substance.

7. A metal surface according to claim 6 wherein the substance is a protein.

8. A metal surface according to claim 7 wherein the protein is an antibody.

9. A method of bonding a chemical species to a metal surface, characterised by the steps of

(i) bonding a co-ordinating polymer to a metal surface, the polymer having a first set of functional groups capable of bonding to the surface and having a second set of functional groups capable of bonding to the chemical species, the groups of the first set being pendant from the polymer chain or forming part thereof, the second set being such that the groups thereof do not bond to the metal surface; and

(ii) bonding the chemical species to the polymer via the second set of functional groups.

10. A method according to claim 9 wherein the coordinating polymer is water-soluble and step (i) is carried out by contacting an aqueous solution thereof with the metal surface, and wherein the chemical species is a biologically active, water-soluble macromolecular substance and step (ii) is carried out by contacting the metal surface to which the polymer has been bonded with an aqueous solution of the chemical species.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-1 500 127 (ABBOTT LABORATORIES) * Page 1, line 42 - page 2, line 31; page 2, line 50 - page 3, line 16; page 9, lines 15-21 * | 1,2,4,6 -10 | G 01 N 33/553 G 01 N 33/543 G 01 N 33/547 |
| X | WO-A-8 603 837 (IQ (BIO) LTD) * Page 6, lines 10-17; figures 1,2 * | 1,2,4,6 -10 | |
| X | EP-A-0 254 575 (ARES-SERONO RESEARCH & DEVELOPMENT LTD PARTNERSHIP) * Page 4, lines 12-15,42-49; page 5, table 1; page 7, lines 60-61; page 8, lines 17-18 * | 1,2,4,6 -10 | |
| Y | US-A-3 979 184 (I. GIAEVER) * Figure 1; column 5, line 46 - column 6, line 10 * | 1-10 | |
| Y | GB-A-2 136 953 (W.K. WANG) * Figure 2; column 1, lines 54-67 * | 1-10 | |

TECHNICAL FIELDS
SEARCHED (Int. Cl.4)

G 01 N

The present search report has been drawn up for all claims

| Place of search | Date of completion of the search | Examiner |
|---|---|---|
| THE HAGUE | 11-08-1989 | VAN BOHEMEN C.G. |

EPO FORM 1503 03.82 (P0401)